# EUROPEAN PATENT SPECIFICATION

(11) **EP 0 471 833 B1**
(45) Date of publication and mention of the grant of the patent: **14.06.1995**
(21) Application number: 91906462.6
(22) Date of filing: 06.03.1991
(51) Int. Cl.: C07K 5/06

(54) **METHOD FOR INCREASING THE ALPHA TO BETA RATIO IN AN ASPARTAME COUPLING REACTION**
VERFAHREN ZUR ERHÖHUNG DES ALPHA/BETA-VERHÄLTNISSES IN EINER ASPARTAMKUPPLUNGSREAKTION
PROCEDE POUR AUGMENTER LA PROPORTION D'ISOMERES ALPHA PAR RAPPORT AUX ISOMERES BETA DANS UNE REACTION DE COUPLAGE DE L'ASPARTAME

(30) Priority: 12.03.1990 US 492524
(43) Date of publication of application: 26.02.1992
(73) Proprietor: THE NUTRASWEET COMPANY, Deerfield, IL 60015 (US)
(72) Inventor: GELMAN, Yefim, Arlington Heights, IL 60004 (US)
(74) Representative: Blum, Rudolf Emil Ernst
(86) International application number: US9101527
(87) International publication number: WO9113860

(56) References cited:
- WO-A-83/01619
- FR-A- 2 558 838
- US-A- 3 933 781
- US-A- 4 745 210
- US-A- 4 760 164
- US-A- 4 810 816
- US-A- 4 820 861
- US-A- 4 946 988

## Description

This application is a continuation-in-part application of U.S. Serial No. 07/219,613, filed on July 14, 1988, which is incorporated by reference herein.

### BACKGROUND OF THE INVENTION

This invention relates to methods of manufacturing L-aspartyl-L-phenylalanine methyl ester hydrochloride (APM(HCl)), which is used to prepare alpha-L-aspartyl-L-phenylalanine methyl ester (α-APM), a sweetening agent which is approximately 200 times sweeter than sucrose. More particularly, this invention relates to methods for increasing the ratio of α to β isomers in a process for manufacturing L-aspartyl-L-phenylalanine methyl ester hydrochloride.

In a conventional method for manufacturing α-APM, L-phenylalanine and N-formyl aspartic anhydride are coupled in the presence of acetic acid to yield an isomeric mixture of α,β-N-formyl-L-aspartyl-L-phenylalanine. This mixture is subsequently deformylated and esterified to produce α-APM(HCl), which is used to prepare α-APM.

In conventional manufacturing methods, the coupling of L-phenylalanine and N-formyl aspartic anhydride produces an isomeric mixture having an α to β ratio of about 3.0-3.8 to 1. This coupling typically takes place in the presence of 11-16 moles of acetic acid per mole of L-phenylalanine as a solvent.

U.S. 3,933,781 discloses a process with up to 24 moles of solvent per mole of L-phenylalanine, however without a significant improvement of the α to β ratio, which is disclosed to be about 4.

U.S. 4,820,861 discloses a continuous production process using at least two solvents, one of which is a carboxylic acid, which gives improved α to β ratios if compared with a corresponding batch process.

Because the β isomer is not sweet, it must be removed from the mixture. Thus, it is desirable to have a minimal amount of the β isomer. However, attempts to minimize the amount of β isomer must not make the process cost inefficient.

Conventionally, the coupling must be carried out at elevated temperatures so that the reaction slurry does not solidify. However, the elevated temperature decreases the selectivity of the reaction, which reduces the α/β ratio.

Thus, an improved yield of the α-isomer in the coupling step of an APM manufacturing batch process is desired. The present invention teaches such a batch process which produces such an improved yield.

### SUMMARY OF THE INVENTION

The present invention teaches an improved N-formyl aspartic anhydride and L-phenylalanine or its lower alkyl ester coupling step which results in an improved α to β ratio. The coupling step takes place in the presence of one or more solvents and a straight chain or branched C₂-C₁₀ carboxylic acid, preferably acetic acid. Both the addition of one or more solvents and an increased amount of the carboxylic acid improve the ratio of α to β isomer in the product, N-formyl-L-aspartyl-L-phenylalanine or lover alkyl esters thereof.

### DETAILED DESCRIPTION OF THE PREFERRED EMBODIMENT

In a preferred embodiment, the coupling step of an α-APM manufacturing process is conducted in the presence of a C₂-C₁₀ carboxylic acid, preferably acetic acid, in any amount along with one or more additional solvents.

In one APM manufacturing process, a protected L-aspartic anhydride such as N-formyl-L-aspartic anhydride is first produced, typically by formylating L-aspartic acid in a reaction mixture of formic acid and acetic anhydride. The N-formyl-L-aspartic anhydride is then coupled with L-phenylalanine or a lower alkyl ester thereof ("L-Phe"). The reaction step is typically carried out in the presence of acetic acid, which acts as a solvent. The acetic acid has conventionally been added in amounts of 11-16 moles per mole of L-phenylalanine or lower alkyl ester thereof. The coupling step produces an isomeric mixture of α,β-N-formyl-L-aspartyl-L- phenylalanine or lower alkyl ester thereof, with an α- isomeric yield of 65-75% of the mixture considered successful. The isomeric mixture is then subjected to additional steps to produce α-APM hydrochloride. The mixture is deblocked by adding an effective amount of hydrochloric acid. Residual acetic acid and an acid such as formic acid are then removed from the mixture. The deformylated isomers are then esterified by addition of an effective amount of methanol, water, and hydrochloric acid to yield α- and β- APM hydrochloride. The α-APM hydrochloride is then precipitated out of the mixture. The β- isomer, which is present in relatively high amounts, can then be recovered.

It has been discovered that the α- to β- isomer ratio, and thus, the resulting yield, can be increased during the step in which N-formyl-L-aspartic anhydride and L-phenylalanine are coupled.

The yield can be improved by using at least two different solvents, one of which is a C₂-C₁₀ carboxylic acid, preferably acetic acid. An amount of two or more solvents including a carboxylic acid similar to or greater than that conventionally used (11-16 moles per mole of Phe or lower alkyl ester thereof) is preferred. It has surprisingly been found that a minimal amount of an additional solvent is effective to significantly increase the α to β ratio of the coupling product. It has also surprisingly been found that the α to β ratio continues to increase as the amount of solvents used increases.

The process works equally well in the coupling of benzyloxycarbonyl-aspartic anhydride and L-phenylalanine or lower alkyl esters thereof. This process is especially preferred in a coupling process with L-phenylalanine methyl ester. The benzyloxycarbonyl group is removed by hydrogenation and the α-isomer is similarly precipitated.

Any organic solvent may be used as an additional solvent in the process. The solvents may be selected from the group including, but not limited to hydrocarbons, aromatic hydrocarbons, halocarbons, secondary or tertiary alcohols, ketones, esters, ethers, lover alkyl phosphates, and nitriles. One of the additional solvents is preferably an ether. The additional solvent or solvents used with the carboxylic acid are preferably selected from the group including, but not limited to methyl tert-butyl ether, ethyl tert-butyl ether, toluene, xylene, cymene, ether, anisole, furfurylacetate, methyl ethyl ketone, 2-propanol, and triethyl phosphate. Methyl tert-butyl ether is an especially preferred solvent. The solvents may be added in any amount relative to each other to produce an increased molar ratio. A preferred range of carboxylic acid to second solvent molar ratios is 1:10 to 25:1. An especially preferred range of carboxylic acid to second solvent molar ratio is 1:2 to 2:1.

The minimum amount of total solvent added is preferably 10 moles per mole of L-phenylalanine or lower alkyl ester thereof, with no real maximum limitation. In fact, increased amounts of the solvents show increased yield of the α-isomer. However, it may become cost-ineffective or process facilities may not be large enough to accommodate large quantities of solvent. A preferred total solvent amount is 144 to 723 moles of total solvent per mole of L-phenylalanine or lower alkyl ester thereof. Especially preferred is a combination of 76.8 moles of MTBE and 76.8 moles of acetic acid per mole of L-phenylalanine.

The coupling reaction may be carried out at temperatures as low as -15°C. This low temperature apparently reduces the rate of reaction which results in an additional increase in the α to β ratio of the coupling product. A preferred temperature range is -5°C to 50°C.

The following examples detail the effects of the use of additional solvents on the α- to β- isomer ratio of the N-formyl-L-aspartyl L- phenylalanine and the yield of the α-product.

### EXAMPLE 1--DILUTED COUPLING OF N-FORMYL-L-ASPARTIC ANHYDRIDE AND L-PHENYLALANINE (ACETIC ACID & SECOND SOLVENT)

In each of the following couplings, 36.6 ml. of acetic acid was added per gram of L-phenylalanine and 80.6 ml. of the second solvent was added per gram of L-phenylalanine. The coupling reaction was carried out for 5 hours at 5°C and 17 hours at 20-22°C.

| Second Solvent | α/β Ratio | α-FAP yield |
|---|---|---|
| Hexane | 3.52 | 77.90 |
| Cyclohexane | 4.36 | 81.35 |
| Benzene | 4.04 | 80.16 |
| Toluene | 6.26 | 86.23 |
| Xylene | 6.02 | 85.80 |
| Mesitylene | 6.34 | 86.37 |
| Tetramethyl benzene | 6.24 | 86.18 |
| Ethyl benzene | 6.36 | 86.42 |
| Diethyl benzene | 6.05 | 85.82 |
| Cumene | 6.05 | 85.82 |
| Cymene | 5.67 | 85.01 |
| Dichloroethylene | 4.12 | 80.45 |
| Chloroform | 4.72 | 82.53 |
| Carbon tetrachloride | 5.89 | 85.49 |
| 2-Propanol | 4.18 | 80.72 |
| Methylethylketone | 4.16 | 80.65 |
| Methyl acetate | 5.21 | 83.90 |
| Ethyl acetate | 3.63 | 78.40 |
| Ether | 6.98 | 87.47 |
| Methyl t-butylether | 8.15 | 89.07 |
| Dimethoxyethylenglycol | 6.14 | 86.00 |
| Diethoxyethylenglycol | 6.41 | 86.51 |
| t-Butoxy-2-methoxyethane | 5.90 | 85.50 |
| t-Butoxy-2-ethoxyethane | 6.92 | 87.40 |
| 2-Methoxyethylether | 4.37 | 81.38 |
| 1.4 Dioxan | 7.17 | 87.80 |
| Tetrahydrofuran | 3.89 | 79.57 |
| Furfuryl acetate | 5.06 | 83.50 |
| Acetonitrile | 3.57 | 78.13 |
| Triethylphosphate | 4.47 | 81.73 |

### EXAMPLE 2- DILUTED COUPLING OF N-FORMYL-L-ASPARTIC ANHYDRIDE AND L-PHENYLALANINE WITH SECOND AND THIRD SOLVENTS

In each of the following couplings, 36.6 ml. of acetic acid was added per gram of L-phenylalanine, and 40.3 ml. of the second and third solvents was added per gram of L-phenylalanine. The reaction temperature was 5°C for 5 hours and 20-22°C for 17 hours.

| Second solvent | Third Solvent | α/β Ratio | α-FAP Yield |
|---|---|---|---|
| 1.4 Dioxane | Toluene | 8.22 | 89.16 |
| 1.4 Dioxane | Xylene | 8.28 | 89.23 |
| Dimethoxyethylenglycol | Toluene | 6.70 | 87.02 |
| Dimethoxyethylenglycol | Xylene | 5.96 | 85.64 |
| Dimethoxyethylenglycol | Cymene | 6.34 | 86.37 |
| Diethoxyethylenglycol | Toluene | 7.03 | 87.60 |
| Diethoxyethylenglycol | Xylene | 6.85 | 87.20 |
| Methyl t-butylether | Toluene | 7.46 | 88.18 |
| Methyl t-butylether | Xylene | 7.98 | 88.90 |

### EXAMPLE 3--DILUTED COUPLING OF N-FORMYL-L-ASPARTIC ANHYDRIDE AND L-PHENYLALANINE WITH METHYL TERT-BUTYL ETHER

Acetic acid and methyl tert-butyl ether were added in a variety of concentrations to a coupling reaction of L-phenylalanine and N-formyl-L-aspartic anhydride at 20°C. The mixture was stirred at this temperature for 30 minutes, then agitation was stopped and the mixture was left at 20°C for 3 hours. The crystals of the isomeric mixture of the reaction product precipitated out and the slurry was agitated for two more hours and a sample of the slurry was taken.

| Molar Ratio AcOH/L-Phe | Molar Ratio MTBE/L-Phe | α-N-formyl-aspartyl-phenylalanine | α/β Ratio |
|---|---|---|---|
| 11.1/1 | 1.79/1 | 79.5 | 3.87 |
| 11.1/1 | 2.73/1 | 79.9 | 3.97 |
| 11.1/1 | 5.47/1 | 84.5 | 5.42 |
| 11.1/1 | 10.93/1 | 86.3 | 6.57 |

### EXAMPLE 4--COUPLING OF N-FORMYL-L- ASPARTIC ANHYDRIDE AND PHENYLALANINE METHYL ESTER (ACETIC ACID & METHYL TERT-BUTYL ETHER)

L-phenylalanine methyl ester (3 g., 0.017 moles) was dissolved in methyl tert-butyl ether (164 ml., 1.410 moles) and 82 ml. (1.435 moles) of acetic acid. This solution was cooled and 2.66 g. (0.0186 moles) of N-formyl-L-aspartic anhydride was added. The mixture was stirred at 5°C for 5 hours. The resulting N-formyl-L-aspartyl-L-phenylalanine methyl ester mixture contained 88.3% of the α- isomer and 11.7% of the β- isomer.

### EXAMPLE 5--COUPLING OF N-BENZYLOXYCARBONYL L-ASPARTIC ANHYDRIDE AND PHENYLALANINE METHYL ESTER (ACETIC ACID & METHYL TERT-BUTYL ETHER)

N-Benzyloxycarbonyl aspartic anhydride (4.33 g., 0.0174 moles) was added to a mixture of methyl tert-butyl ether (150 ml., 1.292 moles) and acetic acid (82 ml., 1.435 moles). This mixture was cooled to 5°C. A solution of 3 g. (0.0167 moles) of L-phenylalanine methyl ester and 14 ml. (0.12 moles) of methyl tert-butyl ether was added. The resulting mixture was stirred for 3 hours at 5°C. The resulting N-Benzyloxycarbonyl-L-aspartyl L-phenylalanine methyl ester mixture contained 83.9% of the α-isomer and 16.1% of the β- isomer.

### EXAMPLE 6--COUPLING OF N-FORMYL-L-ASPARTIC ANHYDRIDE AND L-PHENYLALANINE (PROPIONIC ACID AND METHYL TERT-BUTYL ETHER)

L-phenylalanine (2.73 g., 0.0165 moles) was added to a mixture of propionic acid (100 ml., 0.9 moles) and methyl tert-butyl ether (200 ml., 1.72 moles). The resulting mixture was cooled to 5°C and 2.65 g. (0.019 moles) of N-formyl-L-aspartic anhydride was added. The mixture was stirred at 5°C for 5 hours. The resulting N-formyl-L-aspartyl-L-phenylalanine yielded 86.8% of the α- isomer and 13.2% of the β- isomer.

## Claims

1. A method of producing a mixture of the α and β isomers of N- protected L-aspartyl-L-phenylalanine or lower alkyl ester thereof comprising:
coupling a protected aspartic anhydride and L-phenylalanine or a lower alkyl ester thereof in the presence of a first solvent comprising a C₂-C₁₀ carboxylic acid and at least one additional solvent selected from the group consisting of hydrocarbons, aromatic hydrocarbons, halocarbons, secondary alcohols, tertiary alcohols, ketones, esters, ethers, lower alkyl phosphates and nitriles, the ratio of carboxylic acid to said additional solvent being in the range of 1:10 and 25:1 and the total solvent molar amount comprising 144 to 723 moles of solvent per mole of L-phenylalanine or lower alkyl ester thereof.

2. The method of Claim 1 comprising the addition of at least two additional solvents.

3. The method of Claim 1 wherein said carboxylic acid is acetic acid.

4. The method of Claim 1 wherein said carboxylic acid is propionic acid.

5. The method of Claim 1 wherein said additional solvent is selected from the group consisting of methyl tert-butyl ether, ethyl tert-butyl ether, hexane, cyclohexane, benzene, toluene, xylene, mesitylene, tetramethyl benzene, ethyl benzene, diethyl benzene, cumene, cymene, dichloroethylene, chloroform, carbon tetrachloride, 2-propanol, methylethyl ketone, methyl acetate, ethyl acetate, ether, dimethoxyethylenglycol, diethoxyethylenglycol, t-butoxy-2-methoxyethane, t-butoxy-2-ethoxyethane, 2-methoxyethylether, 1.4 dioxane, tetrahydrofuran, furfuryl acetate, acetonitrile, and triethylphosphate.

6. The method of Claim 1 wherein said additional solvent comprises methyl tert-butyl ether.

7. The method of Claim 1 wherein said lower alkyl ester of L-phenylalanine comprises L-phenylalanine methyl ester.

8. The method of Claim 1 wherein the ratio of acetic acid to additional solvent is in the range between 1:2 and 2:1.

9. The method of Claim 1 wherein the total solvent amount comprises about 154 to 308 moles of solvent per mole of L-phenylalanine or lower alkyl ester thereof.

10. The method of Claim 1 wherein said coupling reaction is carried out at a temperature in the range of -15°C to 50°C.

11. The method of Claim 10 wherein said coupling reaction is carried out at a temperature in the range of -5°C to 25°C.

## Patentansprüche

1. Verfahren zur Herstellung einer Mischung der alpha und beta-Isomeren von N-geschütztem L-Aspartyl-L-phenylalanin oder niedriger Alkylester desselben, umfassend:
Kupplung eines geschützten Asparaginsäureanhydrids und L-Phenylalanin oder eines niedrigen Alkylesters desselben in Anwesenheit eines ersten Lösungsmittels umfassend eine C₂-C₁₀-Carbonsäure und mindestens ein weiteres Lösungsmittel, ausgewählt aus der Gruppe bestehend aus Kohlenwasserstoffen, aromatischen Kohlenwasserstoffen, halogenierten Kohlenwasserstoffen, sekundären Alkoholen, tertiären Alkoholen, Ketonen, Estern, Ethern, niedrigen Alkylphosphaten und Nitrilen, wobei das Verhältnis der Carbonsäure zum besagten zusätzlichen Lösungsmittel im Bereich zwischen 1:10 und 25:1 ist und die gesamte molare Menge 144 bis 723 Mol Lösungsmittel pro Mol L-Phenylalanin oder niedriger Alkylester desselben umfasst.

2. Verfahren gemäß Anspruch 1, umfassend die Zugabe von mindestens zwei zusätzlichen Lösungsmitteln.

3. Verfahren gemäß Anspruch 1, worin die besagte Carbonsäure Essigsäure ist.

4. Verfahren gemäß Anspruch 1, worin die besagte Carbonsäure Propionsäure ist.

5. Verfahren gemäß Anspruch 1, worin das besagte zusätzliche Lösungsmittel ausgewählt ist aus der Gruppe von Methyl tert-butylether, Ethyl tert-butylether, Hexan, Cyclohexan, Benzol, Toluol, Xylol, Mesitylen, Tetramethybenzol, Ethylbenzol, Diethylbenzol, Cumol, Cymol, Dichlorethylen, Chloroform, Tetrachlorkohlenstoff, 2-Propanol, Methylethylketon, Methylacetat, Ethylacetat, Ether, Dimethoxyethylenglycol, Diethoxyethylenglycol, t-Butoxy-2-methoxyethan, t-Butoxy-2-ethoxyethan, 2-Methoxyethylether, 1,4-Dioxan, Tetrahydrofuran, Furfurylacetat, Acetonitril und Triethylphosphat.

6. Verfahren gemäß Anspruch 1, worin das besagte zusätzliche Lösungsmittel Methyl tert-butylether umfasst.

7. Verfahren gemäß Anspruch 1, worin der besagte niedrige Alkylester von L-Phenylalanin, L-Phenylalaninmethylester umfasst.

8. Verfahren gemäß Anspruch 1, worin das Verhältnis von Essigsäure zum zusätzlichen Lösungsmittel im Bereich zwischen 1:2 und 2:1 ist.

9. Verfahren gemäß Anspruch 1, worin die Gesamt-Lösungsmittelmenge zwischen 154 bis 308 mol Lösungsmittel pro mol L-Phenylalanin, oder niedriger Alkylester desselben, umfasst.

10. Verfahren gemäß Anspruch 1, worin die besagte Kupplungsreaktion bei einer Temperatur, im Bereich zwischen -15°C bis 50°C, ausgeführt wird.

11. Verfahren gemäß Anspruch 10, worin die besagte Kupplungsreaktion bei einer Temperatur, im Bereich zwischen -5°C bis 25°C, ausgeführt wird.

## Revendications

1. Un procédé de fabrication d'un mélange d'isomères alpha et bêta du L-aspartyl-L-phénylalanine N-protégé, ou des esters d'alkyle inférieurs de ce-dernier, comprenant:
couplage d'un anhydride aspartique protégé et de L-phénylalanine ou un ester d'alkyle inférieur de cedernier, en présence d'un premier solvant comprenant un acide carboxylique C₂-C₁₀ et au moins un solvant supplémentaire, qui est sélectionné du groupe consistant d'hydrocarbures, d'hydrocarbures aromatiques, d'hydrocarbures halogénés, d'alcools secondaires, d'alcools tertiaires, de cétones, d'esters, d'éthers, de phosphates d'alkyle inférieurs et de nitriles, dont les proportions d'acide carboxylique et de solvant supplémentaires sont dans une fourchette de 1:10 et 25:1 et dont la quantité molaire totale du solvant comprend 144 à 723 moles de solvant par mole de L-phénylalanine ou d'esters d'alkyle inférieurs de ce-dernier.

2. Le procédé de la revendication 1, comprenant l'addition d'au moins deux solvants supplémentaires.

3. Le procédé de la revendication 1, dans lequel ledit acide carboxylique est l'acide acétique.

4. Le procédé de la revendication 1, dans lequel ledit acide carboxylique est l'acide propionique.

5. Le procédé de la revendication 1, dans lequel ledit solvant supplémentaire est sélectionné du groupe consistant du méthyl tert.-butyl éther, l'éthyl tert.-butyl éther, l'hexane, le cyclohexane, le benzène, le toluène, le xylène, le mésitylène, le tetraméthyl benzène, l'éthyl benzène, le diéthylbenzène, le cumène, le cymène, le dichloréthylène, le chloroforme, le tetrachlorure de carbone, l'éther, l'acétate de méthyle, l'acétate d'éthyle, le diméthoxyéthylèneglycol, le diéthoxyéthylèneglycol, le t-butoxy-2-méthoxyéthane, le t-butoxy-2-éthoxyéthane, le 2-méthoxyéthyléther, le 1,4-dioxane, le tétrahydrofurane, l'acétonitrile, l'acétate furfurylique, le méthyl éthyl cétone, 2-propanole et le triéthylphosphate.

6. Le procédé de la revendication 1, dans lequel ledit solvant supplémentaire est le méthyl tert.-butyl éther.

7. Le procédé de la revendication 1, dans lequel ledit ester d'alkyle inférieur de L-phénylalanine comprend l'ester de méthyle de L-phénylalanine.

8. Le procédé de la revendication 1, dans lequel les proportions d'acide acétique au solvant supplémentaire sont dans une fourchette de 1:2 et 2:1.

9. Le procédé de la revendication 1, dans lequel la quantité totale du solvant comprend environ 154 à 308 moles de solvant par mole de L-phénylalanine ou d'esters d'alkyle inférieurs de ce-dernier.

10. Le procédé de la revendication 1, dans lequel ladite réaction de couplage est effectuée à une température comprise entre -15°C à 25°C.

11. Le procédé de la revendication 10, dans lequel ladite réaction de couplage est effectuée à une température comprise entre -5°C à 25°C.
